# EUROPEAN PATENT SPECIFICATION

(11) **EP 4 292 454 B1**
(45) Date of publication and mention of the grant of the patent: **02.04.2025**
(21) Application number: 23208020.0
(22) Date of filing: 18.11.2016
(51) Int. Cl.: A24F 40/30, A24F 40/42, A24F 40/10, A24F 40/20, A24F 40/46

(54) **ELECTRICALLY-POWERED AEROSOL DELIVERY SYSTEM**
ELEKTRISCH ANGETRIEBENES AEROSOLABGABESYSTEM
SYSTÈME DE DISTRIBUTION D'AÉROSOL À ALIMENTATION ÉLECTRIQUE

(30) Priority: 24.11.2015 US 201514950724
(43) Date of publication of application: 20.12.2023
(62) Divisional of application: 22168595.1
(73) Proprietor: RAI Strategic Holdings, Inc., Winston-Salem, NC 27101 (US)
(72) Inventor: SEARS, Stephen Benson, Siler City, 27344 (US); TALUSKIE, Karen V., Winston-Salem, 27104 (US); DAVIS, Michael F., Clemmons, 27012 (US); ADEME, Balager, Winston-Salem, 27127 (US); HUBBARD, Sawyer Austin, Winston-Salem, 27104 (US)
(74) Representative: D Young & Co LLP

(56) References cited:
- WO-A2-2011/117759
- CA-A1- 2 940 842
- US-A1- 2014 261 486
- US-A1- 2014 299 125
- US-A1- 2015 027 454
- US-A1- 2015 027 469
- US-A1- 2015 257 447

## Description

### BACKGROUND

### Field of the Disclosure

The present disclosure relates to aerosol delivery devices and systems, such as smoking articles; and more particularly, to aerosol delivery devices and systems that utilize electrically-generated heat for the production of aerosol (e.g., smoking articles commonly referred to as electronic cigarettes). The aerosol delivery devices and systems may be configured to heat an aerosol precursor, which incorporates materials that may be, though not necessarily, made or derived from tobacco or otherwise incorporate tobacco, and which are capable of vaporizing to form an inhalable aerosol for human consumption.

### Description of Related Art

Many smoking devices have been proposed through the years as improvements upon, or alternatives to, smoking products that require combusting tobacco for use. Many of those devices purportedly have been designed to provide the sensations associated with cigarette, cigar, or pipe smoking, but without delivering considerable quantities of incomplete combustion and pyrolysis products that result from the burning of tobacco. To this end, there have been proposed numerous smoking products, flavor generators, and medicinal inhalers that utilize electrical energy to vaporize or heat a volatile material, or attempt to provide the sensations of cigarette, cigar, or pipe smoking without burning tobacco to a significant degree. See, for example, the various alternative smoking articles, aerosol delivery devices and heat generating sources set forth in the background art described in U.S. Pat. No. 7,726,320 to Robinson et al.; and U.S. Pat. App. Pub. Nos. 2013/0255702 to Griffith, Jr. et al.; and 2014/0096781 to Sears et al. See also, for example, the various types of smoking articles, aerosol delivery devices and electrically powered heat generating sources referenced by brand name and commercial source in U.S. Pat. App. Ser. No. 14/170,838, filed February 3, 2014, to Bless et al.

It would be desirable to provide an electrically-powered aerosol delivery system that is capable of allowing the user thereof to draw aerosol that is highly flavorful. It may also be desirable for the aerosol to be provided under pleasing or comfortable conditions upon being drawn into the mouth of the user.

See also the aerosol delivery systems described in US2014/261486, WO2011/117759, US2015/257447, US2015/027454, US2015/027469, CA2940842 and US2014/299125. Document US2014/261486 describes a vapor-enhancing apparatus for an electronic vapor smoking article, where a vapor-enhancing element may provide flavor enhancement for the aerosol-generating elements. The vapor-enhancing element enhances the vapor drawn through the filter material, the vapor-enhancing element comprising one of a plurality of objects deposited within the filter material, a thread, a filament, a microcapsule, a capsule, a pellet, a granule, a flavorant, a plurality of serially-engaged objects, and combinations thereof.

### SUMMARY OF THE DISCLOSURE

The present disclosure relates to aerosol delivery systems. Such systems have the ability to generate aerosol as a result of heat generated by electrical power sources, and to deliver aerosol that is intended to be drawn into the mouth of a user. Of particular interest are aerosol delivery systems that provide components of tobacco in an aerosol form, such as is provided to smokers by devices commonly known or characterized as electronic cigarettes. As used herein, the term "aerosol" is meant to include vapors, gases, aerosols, and/or particulate matter of a form or type suitable for human inhalation, whether visible or not, and whether or not of a form that might be considered to be "smoke-like."

The above needs are met by means of an aerosol delivery system as defined in the appended set of claims. Such an aerosol delivery system comprises a control body portion, wherein the control body portion includes a first elongate tubular member having opposed ends, and a power source disposed therein. A cartridge body portion includes a second tubular member having opposed first and second ends. The first end of the cartridge body portion is engaged with one of the opposed ends of the control body portion. The cartridge body portion further comprises a first aerosol generation arrangement disposed within the second tubular member, and configured to operably engage the power source upon engagement between the one of the opposed ends of the control body portion and the first end of the cartridge body portion. The second end of the cartridge body portion is further configured as a mouth-engaging end. The cartridge body portion further includes a second aerosol generation arrangement disposed between the first aerosol generation arrangement and the mouth-engaging end of the aerosol delivery system, the second aerosol generation arrangement being either removably engaged with the cartridge body portion or housed within the second tubular member of the cartridge body portion. The second aerosol generation arrangement further includes a plurality of aerosol generation elements, wherein the plurality of aerosol generation elements is selected fromone or more of flavor-releasing particles, flavor-containing and releasing polymers, granules, co-crystals, powders or microcapsules containing tobacco flavors or other substances in a solid, gelatinous, colloidal or gaseous form. The second aerosol generation arrangement further comprises an outer housing body and a plurality of stackable, gas-permeable containers within the outer housing body, each container containing a plurality of aerosol-generating elements

Another aspect of the present disclosure provides a method of forming an aerosol delivery system. Such a method may comprise removably engaging one end of a first elongate tubular member with a first end of a second tubular member, wherein the first elongate tubular member is configured as a control body portion and having a power source disposed therein, and the second tubular member is configured as a cartridge body portion and having a first aerosol generation arrangement disposed therein. The first aerosol generation arrangement is configured to operably engage the power source upon engagement between the one end of the control body portion and the first end of the cartridge body portion. The method comprises inserting of a second aerosol generation arrangement within the second tubular member of the cartridge body portion, between the first aerosol generation arrangement and a second end of the second tubular member, wherein the second end is opposed to the first end and is configured as a mouth-engaging end. In some instances, inserting the second aerosol generation arrangement within the second tubular member may further comprise inserting one or more aerosol generation elements, at least partially forming the second aerosol generation arrangement, into the second tubular member, wherein the one or more (at least one) aerosol generation elements is selected from the group consisting of granules, pellets, beads, discrete small units, carbon pieces, extruded carbon pieces, ceramic beads, marumerized tobacco pieces, extruded or compressed cylindrical or spherical elements, milled tobacco lamina, fillers, flavors, visible aerosol forming materials, binders, ovoid elements, irregularly shaped elements, shredded pieces, flakes, elements including tobacco, elements including a visible aerosol-forming material, adsorbent objects, absorbent objects, capsules, microcapsules, a honeycomb monolith, a single porous structure, and combinations thereof.

These and other features, aspects, and advantages of the present disclosure will be apparent from a reading of the following detailed description together with the accompanying drawings, which are briefly described below. The present disclosure includes any combination of two, three, four, or more of the above-noted aspects as well as combinations of any two, three, four, or more features or elements set forth in this disclosure, regardless of whether such features or elements are expressly combined in a specific embodiment description herein. This disclosure is intended to be read holistically such that any separable features or elements of the present disclosure, in any of its various aspects and embodiments, should be viewed as intended to be combinable unless the context clearly dictates otherwise.

### BRIEF DESCRIPTION OF THE DRAWINGS

Having thus described the disclosure in general terms, reference will now be made to the accompanying drawings, which are not necessarily drawn to scale, and wherein:
FIG. 1 schematically illustrates an aerosol delivery device comprising a cartridge body and a control body, the cartridge body being illustrated in an exploded configuration and the control body being illustrated in an assembled configuration according to an example aspect of the present disclosure;
FIG. 2 schematically illustrates the control body of FIG. 1 in an exploded configuration according to an example aspect of the present disclosure;
FIG. 3 schematically illustrates the cartridge body of FIG. 1 implementing an additional aerosol generation arrangement, including one or more aerosol-generating elements, which does not fall within the scope of the present invention, but is retained for reference;
FIG. 4 schematically illustrates the additional aerosol generation arrangement of FIG. 3, configured as a cartridge including one or more aerosol-generating elements, according to an aspect of the present disclosure;
FIG. 5 schematically illustrates an exploded view of an alternate carbon-based cartridge body according to an example aspect of the present disclosure;
FIG. 6A schematically illustrates an assembled view of the carbon-based cartridge body of FIG. 5, according to an example aspect of the present disclosure;
FIG. 6B schematically illustrates an assembled view of the carbon-based cartridge body, implementing an additional aerosol generation arrangement, including one or more aerosol-generating elements, which does not fall within the scope of the present invention, but is retained for reference;
FIG. 7 is a cross-sectional view of a second aerosol generation arrangement housed within the same outer body as a first aerosol generation arrangement, which does not fall within the scope of the present invention, but is retained for reference;
FIG. 8 is a cross-sectional view of a second aerosol generation arrangement removably attached to the outer body housing a first aerosol generation arrangement, which does not fall within the scope of the present invention, but is retained for reference;
FIG. 9 is a cross-sectional view of a second aerosol generation arrangement comprising multiple, stackable components; and
FIG. 10 is a top view of an aerosol generation arrangement in the form of multiple wedges.

### DETAILED DESCRIPTION OF THE PREFERRED EMBODIMENTS

The present disclosure will now be described more fully hereinafter with reference to exemplary embodiments thereof. These exemplary embodiments are described so that this disclosure will be thorough and complete, and will fully convey the scope of the disclosure to those skilled in the art. Indeed, the disclosure may be embodied in many different forms and should not be construed as limited to the embodiments set forth herein; rather, these embodiments are provided so that this disclosure will satisfy applicable legal requirements. As used in the specification, and in the appended claims, the singular forms "a", "an", "the", include plural variations unless the context clearly dictates otherwise.

As described hereinafter, aspects of the present disclosure relate to aerosol delivery systems. Aerosol delivery systems according to the present disclosure use electrical energy to heat a material (preferably without combusting the material to any significant degree) to form an inhalable substance; and components of such systems have the form of articles most preferably sufficiently compact for such systems to be considered hand-held devices. That is, use of components of preferred aerosol delivery systems does not result in the production of smoke in the sense that aerosol results principally from by-products of combustion or pyrolysis of tobacco, but rather, use of those preferred systems results in the production of vapors (including vapors within aerosols that can be considered to be visible/not visible aerosols that might be considered to be described as smoke-like), resulting from volatilization or vaporization of certain components incorporated therein. In preferred aspects, components of aerosol delivery systems may be characterized as electronic cigarettes, and those electronic cigarettes most preferably incorporate tobacco and/or components derived from tobacco, and hence deliver tobacco derived components in aerosol form.

Aerosol generating pieces of certain preferred aerosol delivery systems may provide many of the sensations (e.g., inhalation and exhalation rituals, types of tastes or flavors, organoleptic effects, physical feel, use rituals, visual cues such as those provided by visible aerosol, and the like) of smoking a cigarette, cigar, or pipe that are provided by lighting and burning tobacco (and hence inhaling tobacco smoke), without any substantial degree of combustion of any component thereof. For example, the user of an aerosol generating piece of the present disclosure can hold and use that piece much like a smoker employs a traditional type of smoking article, draw on one end of that piece for inhalation of aerosol produced by that piece, take or draw puffs at selected intervals of time, and the like.

Aerosol delivery systems of the present disclosure also can be characterized as being suitable vapor-producing articles, aerosol-producing articles, or medicament delivery articles. Thus, such articles, systems, or devices can be adapted so as to provide one or more substances (e.g., flavors, pharmaceutical active ingredients, peptides, protein fragments, and/or protein coats) in an inhalable form or state. For example, inhalable substances can be substantially in the form of a vapor (i.e., a substance that is in the gas phase at a temperature lower than its critical point). Alternatively, inhalable substances can be in the form of an aerosol (i.e., a suspension of fine solid particles or liquid droplets in a gas). For purposes of simplicity, the term "aerosol" as used herein is meant to include vapors, gases, aerosols, and/or particulate matter of a form or type suitable for human inhalation, whether or not visible, and whether or not of a form that might be considered to be smoke-like.

Aerosol delivery systems of the present disclosure most preferably comprise some combination of a power source (i.e., an electrical power source), at least one control component (e.g., means for actuating, controlling, regulating and/or ceasing power supplied for heat generation, such as by controlling electrical current flow from an electrical power release unit to other components of the aerosol generating arrangement), a heater or heat generation component (e.g., an electrical resistance heating element and related components commonly referred to as providing an "atomizer"), and an aerosol precursor composition (e.g., a composition that commonly is a liquid capable of yielding an aerosol upon application of sufficient heat, such as ingredients commonly referred to as "smoke juice," "e-liquid" and "e-juice"), and a mouth end region, mouth-engaging end, or tip for allowing draw upon the aerosol delivery system for aerosol inhalation (e.g., a defined air flow path through the aerosol generation arrangement such that aerosol generated can be withdrawn therefrom upon draw).

More specific formats, configurations and arrangements of components within the aerosol delivery systems of the present disclosure will be evident in light of the further disclosure provided hereinafter. Additionally, the selection and arrangement of various aerosol delivery system components can be appreciated upon consideration of the commercially available electronic aerosol delivery devices, such as those representative products referenced in background art section of the present disclosure.

In some aspects, the use of aerosol delivery devices of the present disclosure may be subjected to many of the physical actions employed by an individual in using a traditional type of smoking article (e.g., a cigarette, cigar or pipe that is employed by lighting and inhaling tobacco). For example, the user of an aerosol delivery device of the present disclosure can hold that article much like a traditional type of smoking article, draw on one end of that article for inhalation of aerosol produced by that article, take puffs at selected intervals of time, or for selected durations of time, etc.

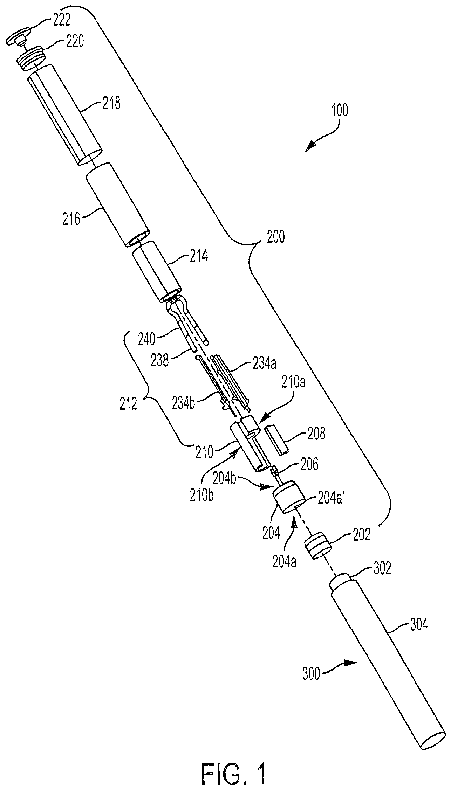

One such example of an aerosol delivery system 100 is illustrated in FIG. 1. **In** particular, FIG. 1 illustrates a partially exploded view of an aerosol delivery system 100 including a cartridge body 200 and a control body 300 (otherwise referred to herein as "cartridge body portion" and "control body portion," respectively). The cartridge body 200 and the control body 300 can be permanently or detachably aligned, or removably engaged, in a functioning relationship. Various mechanisms may be used to connect the cartridge body 200 to the control body 300 to result in a threaded engagement, a press-fit engagement, an interference fit, a magnetic engagement, or the like. The aerosol delivery system 100 may be substantially rod-like, substantially tubular shaped, or substantially cylindrically shaped in some embodiments, when the cartridge body 200 and the control body 300 are in an assembled configuration. As used herein, "tubular" is intended to refer to a hollow, elongated body, but is not limited to a specific cross-sectional shape or to a specific outer contour of the body. One skilled in the art will also appreciate that, in some instances and though not described in detail herein, the cartridge body 200 and the control body 300 forming the aerosol delivery system 100 may be configured in a single-piece, non-detachable form and may incorporate the components, aspects, and features associated with and disclosed in the present disclosure.

In some instances, one or both of the cartridge body 200 and the control body 300 may be referred to as being disposable (i.e., the single piece, non-detachable form previously disclosed) or as being reusable. For example, a reusable control body 300 may have a replaceable battery or a rechargeable battery and thus may be combined with any type of recharging technology, including connection to a typical alternating current electrical outlet, connection to a car charger (i.e., cigarette lighter receptacle), and connection to a computer, such as through a universal serial bus (USB) cable. In general, an aerosol delivery system of the type disclosed herein incorporates a battery or other electrical power source to provide current flow sufficient to provide various functionalities to the article, such as powering of a heater or heating element, powering of control systems, powering of indicators, and the like. The power source can take on various embodiments. Preferably, the power source is able to deliver sufficient power to rapidly heat the heating element to provide for aerosol formation and power the article through use for the desired duration of time. The power source preferably is sized to fit conveniently within the aerosol delivery device/system so that the aerosol delivery device/system can be easily handled; and additionally, a preferred power source is of a sufficiently light weight to not detract from a desirable smoking experience. Further, in some instances, the cartridge body 200 may comprise a single-use cartridge (i.e., disposable), as disclosed, for example, in U.S. Pat. App. Pub. No. 2014/0060555 to Chang et al.

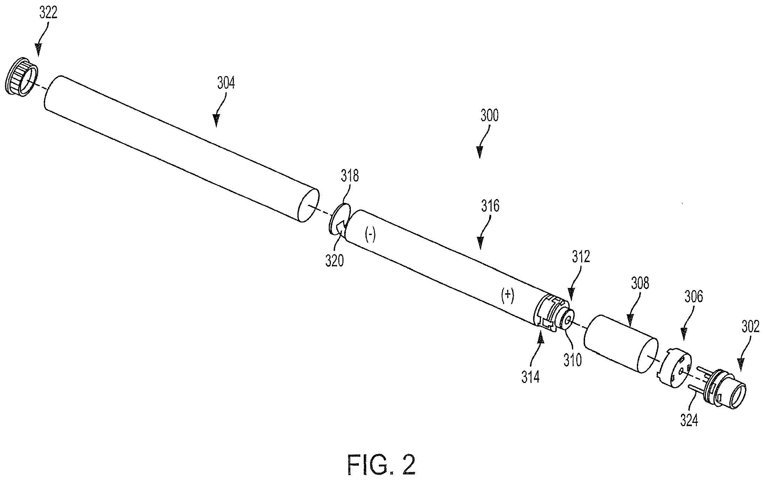

FIG. 2 illustrates an exploded view of the control body 300 of the aerosol delivery system 100 according to another example. As illustrated, the control body 300 may comprise a coupler 302, an outer body 304, a sealing member 306, an adhesive member 308 (e.g., KAPTON^{®} tape), a flow sensor 310 (e.g., a puff sensor or pressure switch), a control component 312, a spacer 314, an electrical power source 316 (e.g., a battery, which may be rechargeable), a circuit board with an indicator 318 (e.g., a light emitting diode (LED)), a connector circuit 320, and an end cap 322. Examples of electrical power sources are described in U.S. Pat. App. Pub. No. 2010/0028766 by Peckerar et al.

With respect to the flow sensor 310, representative current regulating components and other current controlling components including various microcontrollers, sensors, and switches for aerosol delivery devices/systems are described, for example, in U.S. Pat. Nos. 4,735,217 to Gerth et al.; 4,947,874 to Brooks et al.; 5,372,148 to McCafferty et al.; 6,040,560 to Fleischhauer et al.; 7,040,314 to Nguyen et al. and 8,205,622 to Pan; U.S. Pat. Pub. Nos. 2009/0230117 to Fernando et al.; 2014/0060554 to Collett et al.; and 2014/0270727 to Ampolini et al., and U.S. Pat. App. Ser. No 14/209,191, filed March 13, 2014, to Henry et al.

In some instances, the indicator 318 may comprise one or more light emitting diodes. The indicator 318 can be in communication with the control component 312 through the connector circuit 320 and illuminate, for example, during a user drawing on a cartridge body 200 coupled to the coupler 302, as detected by the flow sensor 310. The end cap 322 may be adapted to make visible the illumination provided thereunder by the indicator 318. Accordingly, the indicator 318 may illuminate during use of the aerosol delivery system 100 to simulate the lit end of a smoking article. However, in other instances, the indicator 318 can be provided in varying numbers and can take on different shapes and can even be an opening in the outer body (such as for release of sound when such indicators are present). Additional representative types of components that yield visual cues or indicators, such as light emitting diode (LED) components, and the configurations and uses thereof, are described in U.S. Pat. Nos. 5,154,192 to Sprinkel et al.; 8,499,766 to Newton and 8,539,959 to Scatterday; and U.S. Pat. App. Ser. No. 14/173,266, filed February 5, 2014, to Sears et al.

Still further features, controls or components that can be incorporated into aerosol delivery devices and systems of the present disclosure are described in U.S. Pat. Nos. 5,967,148 to Harris et al.; 5,934,289 to Watkins et al.; U.S. Pat. No. 5,954,979 to Counts et al.; 6,040,560 to Fleischhauer et al.; 7,726,320 to Robinson et al.; 8,365,742 to Hon; 8,402,976 and 8,689,804 to Fernando et al.; U.S. Pat. App. Pub. Nos. 2013/0192623 to Tucker et al.; 2013/0298905 to Leven et al.; 2013/0180553 to Kim et al. and 2014/0000638 to Sebastian et al.; and U.S. Pat. App. Pub. Nos. 2014/0261495 to Novak, III et al. and 2014/0261408 to DePiano et al.

Returning to FIG. 1, the cartridge body 200 is illustrated in an exploded configuration. As illustrated, the cartridge body 200 may comprise a base shipping plug 202, a base 204, a control component terminal 206, an electronic control component 208, a flow tube 210, an atomizer 212, a reservoir substrate 214, an outer body 216, a label 218, a mouthpiece 220, and a mouthpiece shipping plug 222 according to an example embodiment of the present disclosure. The base 204 may be coupled to a first end of the outer body 216 and the mouthpiece 220 may be coupled to an opposing second end of the outer body 216 to enclose the remaining components of the cartridge body 200 therein. The base 204 may be configured to removably engage the coupler 302 of the control body 300. **In** some instances, the base 204 may comprise anti-rotation features that substantially prevent relative rotation between the cartridge body and the control body as disclosed in U.S. Pat. App. Pub. No. 2014/0261495 to Novak, III et al. Various representative coupling mechanisms for upstream and downstream components of electronic cigarettes have been set forth in the patent literature and have been employed for the production of commercially available electronic cigarettes. For example, representative types of coupling mechanisms and components for electronic cigarettes are described in U.S. Pat. App. Pub. No. 2014/0261495 to Novak, III et al., and U.S. Pat. App. Ser. No. 14/170,838, filed February 3, 2014, to Bless et al.

The base shipping plug 202 may be configured to engage and protect the base 204 prior to use of the cartridge body 200. Similarly, the mouthpiece shipping plug 222 may be configured to engage and protect the mouthpiece 220 prior to use of the cartridge body 200. The control component terminal 206, the electronic control component 208, the flow tube 210, the atomizer 212, and the reservoir substrate 214 (engaging the aerosol precursor composition or substance) may be retained within the outer body 216. The label 218 may at least partially surround the outer body 216 and include information such as a product identifier thereon.

Alignment of the components within either or both of the control body and the cartridge body of the aerosol delivery device/system can vary. **In** particular aspects, the aerosol precursor composition can be located near one end of the overall article (e.g., within a cartridge body, which in certain circumstances can be replaceable and disposable), which may be configured to be positioned in relatively closer proximity to the mouth of a user so as to maximize aerosol delivery to the user. Other configurations, however, are not excluded. Generally, the heating element can be positioned sufficiently near the aerosol precursor composition so that heat from the heating element can volatilize the aerosol precursor (and/or one or more flavorants, medicaments, or the like that may likewise be provided for delivery to a user) and form an aerosol for delivery to the user. When the heating element heats the aerosol precursor composition, an aerosol is formed, released, or generated in a physical form suitable for inhalation by a consumer. It should be noted that the foregoing terms are meant to be interchangeable such that reference to release, releasing, releases, or released includes form or generate, forming or generating, forms or generates, and formed or generated. Specifically, an inhalable substance is released in the form of a vapor or aerosol or mixture thereof. Additionally, the selection of various aerosol delivery device components can be appreciated upon consideration of the commercially available electronic aerosol delivery devices, such as those representative products listed above in the present disclosure.

The atomizer (i.e., an aerosol generation arrangement) 212 may comprise a first heating terminal 234a and a second heating terminal 234b, a liquid transport element 238 and a heating element 240. In this regard, the reservoir and/or reservoir substrate 214 may be configured to hold an aerosol precursor composition. The aerosol precursor composition, also referred to as a vapor precursor composition, may comprise a variety of components in different aspects. Such components may include, by way of example, any of a polyhydric alcohol (e.g., glycerin, propylene glycol, or a mixture thereof), nicotine, tobacco, tobacco extract, water, flavorants, and combinations thereof.

The aerosol precursor, or vapor precursor composition, can vary. Most preferably, the aerosol precursor composition is comprised of a combination or mixture of various ingredients or components. The selection of the particular aerosol precursor components, and the relative amounts of those components used, may be altered in order to control the overall chemical composition of the mainstream aerosol produced by the aerosol generation arrangement(s). Of particular interest are aerosol precursor compositions that can be characterized as being generally liquid in nature. For example, representative generally liquid aerosol precursor compositions may have the form of liquid solutions, viscous gels, mixtures of miscible components, or liquids incorporating suspended or dispersed components. Typical aerosol precursor compositions are capable of being vaporized upon exposure to heat under those conditions that are experienced during use of the aerosol generation arrangement(s) that are characteristic of the present disclosure; and hence are capable of yielding vapors and aerosols that are capable of being inhaled.

For aerosol delivery systems that are characterized as electronic cigarettes, the aerosol precursor composition most preferably incorporates tobacco or components derived from tobacco. In one regard, the tobacco may be provided as parts or pieces of tobacco, such as finely ground, milled or powdered tobacco lamina. In another regard, the tobacco may be provided in the form of an extract, such as a spray dried extract that incorporates many of the water soluble components of tobacco. Alternatively, tobacco extracts may have the form of relatively high nicotine content extracts, which extracts also incorporate minor amounts of other extracted components derived from tobacco. In another regard, components derived from tobacco may be provided in a relatively pure form, such as certain flavoring agents that are derived from tobacco. In one regard, a component that is derived from tobacco, and that may be employed in a highly purified or essentially pure form, is nicotine (e.g., pharmaceutical grade nicotine).

As noted above, highly purified tobacco-derived nicotine (e.g., pharmaceutical grade nicotine having a purity of greater than 98% or greater than 99%) or a derivative thereof can be used in the present invention. Representative nicotine-containing extracts can be provided using the techniques set forth in U.S. Pat. No. 5,159,942 to Brinkley et al*.* In certain embodiments, the products of the invention can include nicotine in any form from any source, whether tobacco-derived or synthetically-derived. Nicotinic compounds used in the products of the invention can include nicotine in free base form, salt form, as a complex, or as a solvate. See, for example, the discussion of nicotine in free base form in U.S. Pat. Pub. No. 2004/0191322 to Hansson. At least a portion of the nicotinic compound can be employed in the form of a resin complex of nicotine where nicotine is bound in an ion exchange resin such as nicotine polacrilex. See, for example, U.S. Pat. No. 3,901,248 to Lichtneckert et al. At least a portion of the nicotine can be employed in the form of a salt. Salts of nicotine can be provided using the types of ingredients and techniques set forth in U.S. Pat. No. 2,033,909 to Cox et al. and Perfetti, Beitrage Tabakforschung Int., 12, 43-54 (1983). Additionally, salts of nicotine have been available from sources such as Pfaltz and Bauer, Inc. and K&K Laboratories, Division of ICN Biochemicals, Inc. Exemplary pharmaceutically acceptable nicotine salts include nicotine salts of tartrate (e.g., nicotine tartrate and nicotine bitartrate), chloride (e.g., nicotine hydrochloride and nicotine dihydrochloride), sulfate, perchlorate, ascorbate, fumarate, citrate, malate, lactate, aspartate, salicylate, tosylate, succinate, pyruvate, and the like; nicotine salt hydrates (e.g., nicotine zinc chloride monohydrate), and the like. In certain embodiments, at least a portion of the nicotinic compound is in the form of a salt with an organic acid moiety, including, but not limited to, levulinic acid as discussed in U.S. Pat. Pub. No. 2011/0268809 to Brinkley et al.

The aerosol precursor composition may also incorporate so-called "aerosol forming materials." Such materials may, in some instances, have the ability to yield visible (or not visible) aerosols when vaporized upon exposure to heat under those conditions experienced during normal use of aerosol generation arrangement(s) that are characteristic of the present disclosure. Such aerosol forming materials include various polyols or polyhydric alcohols (e.g., glycerin, propylene glycol, and mixtures thereof). Aspects of the present disclosure also incorporate aerosol precursor components that can be characterized as water, saline, moisture or aqueous liquid. During conditions of normal use of certain aerosol generation arrangement(s), the water incorporated within those aerosol generation arrangement(s) can vaporize to yield a component of the generated aerosol. As such, for purposes of the current disclosure, water that is present within the aerosol precursor composition may be considered to be an aerosol forming material.

It is possible to employ a wide variety of optional flavoring agents or materials that alter the sensory character or nature of the drawn mainstream aerosol generated by the aerosol delivery system of the present disclosure. For example, such optional flavoring agents may be used within the aerosol precursor composition or substance to alter the flavor, aroma and organoleptic properties of the aerosol. Certain flavoring agents may be provided from sources other than tobacco. Exemplary flavoring agents may be natural or artificial in nature, and may be employed as concentrates or flavor packages.

Exemplary flavoring agents include vanillin, ethyl vanillin, cream, tea, coffee, fruit (e.g., apple, cherry, strawberry, peach and citrus flavors, including lime and lemon), maple, menthol, mint, peppermint, spearmint, wintergreen, nutmeg, clove, lavender, cardamom, ginger, honey, anise, sage, cinnamon, sandalwood, jasmine, cascarilla, cocoa, licorice, and flavorings and flavor packages of the type and character traditionally used for the flavoring of cigarette, cigar and pipe tobaccos. Syrups, such as high fructose corn syrup, also can be employed. Certain flavoring agents may be incorporated within aerosol forming materials prior to formulation of a final aerosol precursor mixture (e.g., certain water soluble flavoring agents can be incorporated within water, menthol can be incorporated within propylene glycol, and certain complex flavor packages can be incorporated within propylene glycol). However, in some aspects of the present disclosure, the aerosol precursor composition is free of any flavorants, flavor characteristics or additives.

Aerosol precursor compositions also may include ingredients that exhibit acidic or basic characteristics (e.g., organic acids, ammonium salts or organic amines). For example, certain organic acids (e.g., levulinic acid, succinic acid, lactic acid, and pyruvic acid) may be included in an aerosol precursor formulation incorporating nicotine, preferably in amounts up to being equimolar (based on total organic acid content) with the nicotine. For example, the aerosol precursor may include about 0.1 to about 0.5 moles of levulinic acid per one mole of nicotine, about 0.1 to about 0.5 moles of succinic acid per one mole of nicotine, about 0.1 to about 0.5 moles of lactic acid per one mole of nicotine, about 0.1 to about 0.5 moles of pyruvic acid per one mole of nicotine, or various permutations and combinations thereof, up to a concentration wherein the total amount of organic acid present is equimolar to the total amount of nicotine present in the aerosol precursor composition. However, in some aspects of the present disclosure, the aerosol precursor composition is free of any acidic (or basic) characteristics or additives.

As one non-limiting example, a representative aerosol precursor composition or substance can include glycerin, propylene glycol, water, saline, and nicotine, and combinations or mixtures of any or all of those components. For example, in one instance, a representative aerosol precursor composition may include (on a weight basis) about 70% to about 100% glycerin, and often about 80% to about 90% glycerin; about 5% to about 25% water, often about 10% to about 20% water; and about 0.1% to about 5% nicotine, often about 2% to about 3% nicotine. In one particular non-limiting example, a representative aerosol precursor composition may include about 84% glycerin, about 14% water, and about 2% nicotine. The representative aerosol precursor composition may also include propylene glycol, optional flavoring agents or other additives in varying amounts on a weight basis. In some instances, the aerosol precursor composition may comprise up to about 100% by weight of any of glycerin, water, and saline, as necessary or desired.

Representative types of aerosol precursor components and formulations also are set forth and characterized in U.S. Pat. No. 7,217,320 to Robinson et al. and U.S. Pat. Pub. Nos. 2013/0008457 to Zheng et al.; 2013/0213417 to Chong et al. and 2014/0060554 to Collett et al. Other aerosol precursors that may be employed include the aerosol precursors that have been incorporated in the VUSE^{®} product by R. J. Reynolds Vapor Company, the BLU^{™} product by Lorillard Technologies, the MISTIC MENTHOL product by Mistic Ecigs, and the VYPE product by CN Creative Ltd. Also desirable are the so-called "smoke juices" for electronic cigarettes that have been available from Johnson Creek Enterprises LLC.

The amount of aerosol precursor that is incorporated within the aerosol delivery system is such that the aerosol generation arrangement(s) provide acceptable sensory and desirable performance characteristics. For example, it is highly preferred that sufficient amounts of aerosol forming material (e.g., glycerin and/or propylene glycol), be employed in order to provide for the generation of a mainstream aerosol (visible or not visible) that in many regards resembles the appearance of tobacco smoke. The amount of the aerosol precursor composition within the aerosol generation arrangement(s) may be dependent upon factors such as the number of puffs desired per aerosol generation arrangement. Typically, the amount of the aerosol precursor composition incorporated within the aerosol delivery system, and particularly within the aerosol generation arrangement(s), is less than about 2 g, generally less than about 1.5 g, often less than about 1 g and frequently less than about 0.5 g.

The reservoir substrate 214 may comprise a plurality of layers of nonwoven fibers formed into the shape of a tube encircling the interior of the outer body 216 of the cartridge body 200. Thus, liquid components, for example, can be sorptively retained by the reservoir substrate 214. The reservoir substrate 214 is in fluid connection with the liquid transport element 238. The liquid transport element 238 may be configured to transport liquid (i.e., the aerosol precursor composition) from the reservoir substrate 214 to the heating element 240 via capillary action. Representative types of substrates, reservoirs or other components for supporting the aerosol precursor composition are described in U.S. Pat. No. 8,528,569 to Newton; and U.S. Pat. App. Pub. Nos. 2014/0261487 to Chapman et al and 2015/0059780 to Davis et al.; and U.S. App. Ser. No. 14/170,838; filed February 3, 2014, to Bless et al. Additionally, various wicking materials, and the configuration and operation of those wicking materials within certain types of electronic cigarettes, are set forth in U.S. Pat. App. Pub. No. 2014/0209105 to Sears et al.

As illustrated, the liquid transport element 238 may be in direct contact with the heating element 240. As further illustrated in FIG. 1, the heating element 240 may comprise a wire defining a plurality of coils wound about the liquid transport element 238. In some instances, the heating element 240 may be formed by winding the wire about the liquid transport element 238 as described in U.S. Pat. App. Pub. No. 2014/0157583 to Ward et al. Further, in some instances, the wire may define variable coil spacing, as described in U.S. Pat. App. Pub. No. 2014/0270730 to DePiano et al. Various materials configured to produce heat when an electrical current is applied thereto may be employed to form the heating element 240. Example materials from which the wire coil may be formed include Kanthal (FeCrAl), Nichrome, molybdenum disilicide (MoSi₂), molybdenum silicide (MoSi), molybdenum disilicide doped with aluminum (Mo(Si,Al)₂), graphite and graphite-based materials; and ceramic (e.g., a positive or negative temperature coefficient ceramic).

However, various other methods may be employed to form the heating element 240, and various other aspects of heating elements may be employed in the atomizer 212. For example, a stamped heating element may be employed in the atomizer, as described in U.S. Pat. App. Pub. No. 2014/0270729 to DePiano et al. Further to the above, additional representative heating elements and materials for use therein are described in U.S. Pat. No. 5,060,671 to Counts et al.; U.S. Pat. No. 5,093,894 to Deevi et al.; U.S. Pat. No. 5,224,498 to Deevi et al.; U.S. Pat. No. 5,228,460 to Sprinkel Jr., et al.; U.S. Pat. No. 5,322,075 to Deevi et al.; U.S. Pat. No. 5,353,813 to Deevi et al.; U.S. Pat. No. 5,468,936 to Deevi et al.; U.S. Pat. No. 5,498,850 to Das; U.S. Pat. No. 5,659,656 to Das; U.S. Pat. No. 5,498,855 to Deevi et al.; U.S. Pat. No. 5,530,225 to Hajaligol; U.S. Pat. No. 5,665,262 to Hajaligol; U.S. Pat. No. 5,573,692 to Das et al.; and U.S. Pat. No. 5,591,368 to Fleischhauer et al. Further, chemical heating may be employed in other aspects. A variety of heater components may also be used in particular aspects of the present aerosol delivery device/system. **In** various instances, one or more microheaters or similar solid state heating elements may be used. Exemplary microheaters that may be utilized are further described herein. Further microheaters and atomizers incorporating microheaters suitable for use in the presently disclosed devices/systems are described in U.S. Pat. App. Pub. No. 2014/0060554 to Collett et al.

The first heating terminal 234a and the second heating terminal 234b (e.g., positive and negative terminals) at the opposing ends of the heating element 240 are configured to form an electrical connection (which may be a removable or detachable connection) with the control body 300 when the cartridge body 200 is connected thereto. Further, when the control body 300 is coupled to the cartridge body 200, the electronic control component 208 may form an electrical connection with the control body 300 through the control component terminal 206. The control body 300 may thus employ the electronic control component 208 to determine whether the cartridge 200 is genuine and/or perform other functions. Further, various examples of electronic control components and functions performed thereby are described in U.S. Pat. App. Pub. No. 2014/0096781 to Sears et al.

During use, a user may draw on the mouthpiece or mouth-engaging end 220 of the cartridge body 200 of the aerosol delivery system 100. This may pull air through an opening in the control body 300 and/or in the cartridge body 200. For example, in one instance, an opening may be defined between the coupler 302 and the outer body 304 of the control body 300, as described in U.S. Pat. App. Pub. No. 2014/0261408 to DePiano et al. However, the flow of air may be received through other parts of the aerosol delivery device/system 100 in other aspects. As noted above, in some aspects the cartridge body 200 may include the flow tube 210. The flow tube 210 may be configured to direct the flow of air received from the control body 300 to the heating element 240 of the atomizer 212.

A sensor in the aerosol delivery device/system 100 (e.g., a puff or flow sensor in the control body 300) may sense the puff. More generally, a sensor or detector may be implemented to control of supply of electric power to the heating element 240 when aerosol generation is desired (e.g., upon draw during use). As such, for example, there is provided a manner or method for turning off the power supply to the heating element 240 when the aerosol generation is not desired during use, and for turning on the power supply to actuate or trigger the generation of heat by the heating element 240 during draw. Additional representative types of sensing or detection mechanisms, structure and configuration thereof, components thereof, and general methods of operation thereof, are described in U.S. Pat. Nos. 5,261,424 to Sprinkel, Jr.; 5,372,148 to McCafferty et al.; and PCT WO 2010/003480 by Flick. When the puff is sensed, the control body 300 may direct current to the heating element 240 through a circuit including the first heating terminal 234a and the second heating terminal 234b. Accordingly, the heating element 240 may vaporize the aerosol precursor composition directed to an aerosolization zone from the reservoir substrate 214 by the liquid transport element 238. Thus, the mouthpiece 220 may allow passage of air and entrained vapor (i.e., the components of the aerosol precursor composition in an inhalable form, for example, as an aerosol) from the cartridge body 200 to a consumer drawing thereon. Various other details with respect to the components that may be included in the cartridge body 200, are provided, for example, in U.S. Pat. App. Pub. No. 2014/0261495 to Novak, III et al.

Various components of an aerosol delivery device/system can be chosen from components described in the art and commercially available. Reference is made for example to the reservoir and heater system for controllable delivery of multiple aerosolizable materials in an electronic smoking article disclosed in U.S. Pat. App. Pub. No. 2014/0000638 to Sebastian et al. Note further that portions of the cartridge body 200 illustrated in FIG. 1 are optional. **In** this regard, by way of example, the cartridge body 200 may not necessarily include the flow tube 210, the control component terminal 206, and/or the electronic control component 208, in some instances.

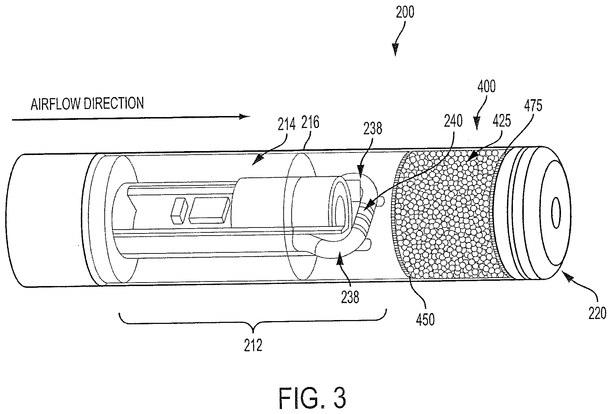

According to FIG. 3, the cartridge body 200 incorporates a second aerosol generation arrangement 400 (the atomizer 212 being considered "a first aerosol generation arrangement") disposed in the outer body 216, longitudinally between the atomizer 212 and the mouthpiece or mouth-engaging end 220 of the cartridge body 200. In some aspects, the second aerosol generation arrangement 400 is generally porous or otherwise configured to allow the passage of air therethrough. In some particular instances, the second aerosol generation arrangement 400 may include one or more aerosol-generating elements 425 that may be comprised of at least one or a plurality of pellets or beads or other appropriate elements or combinations thereof. In some instances, the at least one or a plurality of pellets or beads or other appropriate elements or combinations thereof forming the aerosol-generating element(s) 425 may be coaxially circumscribed by a generally tubular-shaped heat conductive member (not shown), if necessary, and/or circumscribed or otherwise jacketed by insulation (e.g., a non-woven mat or layer of glass filaments or fibers), or other suitable material (not shown).

The overall configuration of the second aerosol generation arrangement 400 within the cartridge body 200 of the aerosol delivery device/system 100 can be considered to be generally cylindrical in nature. Representative preferred beads or other objects may be produced from a formulation that incorporates tobacco (e.g., particulate tobacco), components of tobacco and/or materials that are otherwise derived from tobacco (e.g., tobacco extracts such as aqueous tobacco extracts or nicotine derived from tobacco including pharmaceutical grade nicotine). The beads most preferably incorporate flavors and a visible or non-visible aerosol forming material (e.g., glycerin or other material that generates a visible vapor that resembles smoke). That is, components of the beads are preferably configured to act as substrate components for volatile flavors, vapor forming materials, moisture or other liquid(s), and/or aerosol forming materials that are carried thereby. In some aspects, the aerosol-generating element(s) 425 may include or otherwise comprise or be configured as, for example, marumerized tobacco beads of varying shapes and sizes, a monolith of bonded (e.g., sintered) beads; a porous monolith (e.g., a sponge-like porous structure); a single porous structure; a honeycomb monolith or other porous geometric scaffold; a single piece of a porous material; beads of extruded tobacco; beads of porous material containing tobacco extract (e.g., calcium carbonate, ceramic, glass, or the like); reconstituted tobacco shreds; expanded tobacco shreds; extruded rods of various materials (including hollow cylinders and slotted rods) containing tobacco flavors; shavings, fibers. According to the invention, the second aerosol generation arrangement includes a plurality of aerosol-generating elements selected from one or more of flavor-releasing particles, flavor-containing (and releasing) polymers, granules, co-crystals, capsules, powders, and/or microcapsules of various materials containing tobacco flavors or other substances, whether in a solid, liquid, gelatinous, colloidal, gaseous, or other form; and treatments or combinations thereof.

In certain embodiments, the aerosol-generating element(s) 425 may be at least partially in the form of a coating or film on the walls or lining of the housing body surrounding the aerosol-generating elements. For example, a film comprising a microencapsulated flavorant could be coated on the walls or lining of the housing body.

In general, as used herein, the terms "pellets" and "beads" are meant to include beads, pellets, or other discrete small units or pieces of that may include (in addition to those otherwise disclosed herein), for example, carbon pieces, extruded carbon pieces cut into pellets, ceramic beads, marumerized tobacco pieces, polymer beads, glass beads, and the like, or combinations thereof. For example, granules, pellets or beads can be generally cylindrical or spherical extruded or compressed granules, pellets or beads comprised of a moistened mixture or slurry of milled tobacco lamina, fillers (e.g., granular calcium carbonate), flavors, visible aerosol forming materials and binders (e.g., carboxy methylcellulose) that are formed, cut or spun to the desired size and shape, and then dried to retain the desired configuration. However, such "pellets" or "beads" may comprise any suitable elements, or combination of elements, meeting the preferred aspects as disclosed herein. For example, some or all of the beads or pellets can comprise spherical capsules that are heat sensitive, so that when included in the aerosol-generating element and exposed to heat, the rupture or decomposition thereof causes the release of glycerin, propylene glycol, water, saline, tobacco flavor and/or nicotine or other substances or additives. Also, the beads can comprise ceramic or absorbent clay or silica or absorbent carbon to hold and release an aerosol former. Further, in some aspects, the beads/pellets may comprise a heat conductive material such as, for example, heat conductive graphite, heat conductive ceramic, a metal, tobacco cast on foil, a metal or other suitable material impregnated with appropriate aerosol-generating substances such as glycerin and flavor(s), or a suitable cast sheet material appropriately formed into the desired beads/pellets.

In one particular example, the beads/pellets (particles) may be comprised, by weight, of between about 15% and about 60% of finely milled tobacco particles (e.g., a blend of Oriental, burley and flue-cured tobaccos, essentially all Oriental tobacco, essentially all burley tobacco, or essentially all flue-cured tobacco), between about 15% and about 60% of finely milled particles of calcium carbonate (or finely milled clay or ceramic particles), between about 10% and about 50% of glycerol (and optionally a minor amount of flavors), between about 0.25% and about 15% of a binder (preferably carboxymethylcellulose, guar gum, potassium, or ammonium alginate), and between about 15% and about 50% of water. In another example, the beads/pellets (particles) may be comprised of about 30% of finely milled tobacco particles (e.g., a blend of Oriental, burley and flue-cured tobaccos, essentially all Oriental tobacco, essentially all burley tobacco, or essentially all flue-cured tobacco), about 30% of finely milled particles of calcium carbonate (or finely milled clay or ceramic particles), about 15% of glycerol (and optionally a minor amount of flavors), about 1% of a binder (preferably carboxymethylcellulose, guar gum, potassium, or ammonium alginate), and about 25% of water. In such examples, the particles may be compressed to hold the glycerol and, upon compression, may form a porous matrix that facilitates migration of the aerosol generating components to promote efficient aerosol formation. The manner by which the aerosol forming material is contacted with the substrate material can vary. The aerosol forming material can be applied to a formed material, can be incorporated into processed materials during manufacture of those materials, or can be endogenous to that material. Aerosol-forming material, such as glycerin, can be dissolved or dispersed in an aqueous liquid, or other suitable solvent or liquid carrier, and sprayed onto that substrate material. See, for example, U.S. Patent Appl. Pub. No. 2005/0066986 to Nestor et al. and 2012/0067360 to Conner et al. The calcium carbonate or other inorganic filler assists in creating porosity within the particles, and may also function to absorb heat which may, in some instances limit or otherwise prevent scorching of the aerosol generating components, as well as assisting in and promoting aerosol formation. See also, for example, those types of materials set forth in U.S. Pat. No. 5,105,831 to Banerjee, et al.*,* and U.S. Pat. App. Pub. Nos. 2004/0173229 to Crooks et al.; 2011/0271971 to Conner et al.; and 2012/0042885 to Stone et al.

In certain embodiments, the beads/pellets are in the form of subdivided pieces of an extruded material comprising a particulate material serving as a substrate for flavorants and/or aerosol-forming materials, such as tobacco (e.g., a milled tobacco material) or a filler material (e.g., rice flour or other grain-based material and/or calcium carbonate), one or more binders (e.g., carboxymethylcellulose), one or more flavorants (e.g., menthol), and one or more aerosol-forming materials (e.g., glycerin). Optionally, the beads/pellets may include one or more organic acids (e.g., levulinic acid or lactic acid) and/or one or more sweeteners (a sugar or sugar substitute). The above-noted materials can be mixed together, extruded, and subdivided into pieces of a desired size. Optionally, the mixed materials can also be marumerized.

In one embodiment, the aerosol-generating elements 425, such as those in the form of beads or pellets, can be smoke-treated to impart smoky flavor or aroma. For example, the beads or pellets can be prepared and then subjected to smoke from a combustible source, such as a wood source (e.g., wood selected from hickory, maple, oak, apply, cherry, or mesquite). The beads or pellets can be treated with the smoke for a time sufficient to impart the desired smoky flavor or aroma, with an exemplary time range being about 5 to about 45 minutes. The manner in which the beads or pellets are contacted with smoke can vary, with one example involving heating wood chips in a container until smoke is produced (e.g., heating wood chips to a temperature of about 177-200°F (350-400°F)) and placing the beads or pellets to be treated within a closed environment with the smoke produced by the wood chips.

The composition of the aerosol precursor composition of the first aerosol generation arrangement and the composition of the aerosol-generating elements of the second aerosol generation arrangement are advantageously selected so as to complement one another to produce a desirable sensory experience. In certain embodiments, for example, the nicotine content of the aerosol precursor composition and the aerosol-generating elements are selected such that either or both of the aerosol precursor composition and the aerosol-generating elements may contain nicotine or a nicotinic compound or may be viewed as substantially or completely free of nicotine or a nicotinic compound. In other words, all nicotine content can be within the aerosol-generating elements or all nicotine content can be in the aerosol precursor composition or both compositions can include nicotine in some form.

In some aspects, where the aerosol-generating elements 425 comprise, for example, beads or pellets cast or extruded from materials of the various types set forth above (i.e., a graphite bead including tobacco extract and glycerin), while "damp" or otherwise before drying, may be rolled, for example, between adjacent roller elements, to flatten the shape of the respective beads/pellets. In some instances, the materials of the various types set forth above may be extruded in the form of filamentary strands, wherein the strands may be gathered to form a cylindrical rod or other suitably shaped material (i.e., relative in size to the beads/pellets used to otherwise form the aerosol generation segment) for application in the second aerosol generation arrangement 400. Upon drying, the flattened beads/pellets may then be shredded or otherwise processed to form, for example, strands, flakes, or other filler configuration that is flat or includes a planar segment that inhibits or prevents roll. Any random configurations resulting from the shredding process may be sufficient. In such instances, the flattened and shredded beads/pellets may then be included in the aerosol-generating element(s) 425, and the irregular or random configurations thereof may promote, for instance, a plurality of interstitial air spaces throughout the aerosol-generating element(s) 425, wherein the interstitial air spaces may, in turn, promote heat transfer with the individual objects within the aerosol-generating element(s) 425. That is, heating of the air in the interstitial spaces within the second aerosol generation arrangement 400 may expose more of the aerosol-generating element(s) 425 to the heat from the heating element 240, and thus result in enhanced or otherwise improved heating of the aerosol-generating element(s) 425. In other instances, the heat and the first aerosol (i.e., the combination thereof) produced by the heating element 240 / atomizer 212 are directed through the porous matrix formed by the aerosol-generating element(s) 425, wherein the heated vapors passing through and heating the porous aerosol-generating element(s) 425 promotes, for example, elution (i.e., liquid, fluid, or particulate extraction; steam distillation; etc.) of an enhancement substance (i.e., a flavorant or other additive) from the aerosol-generating element(s) to the first aerosol, or otherwise promotes the enhancement substance being entrained in, imparted to, reacted with, or otherwise interacted with the first aerosol. The interaction between the enhancement substance and the first aerosol may, for example, change or alter the first aerosol, mix the enhancement substance with the first aerosol to form an enhanced aerosol or aerosol mixture, or facilitate a reaction that produces a different aerosol. In such instances, increased interstitial spaces within the aerosol-generating element(s) 425 may promote this interaction process through the second aerosol generation arrangement 400.

In some aspects, the beads / pellets may originate from a tobacco material cast on a foil/paper laminate. More particularly, the tobacco material may comprise, for example, a slurry including reconstituted tobacco, glycerin, and a binder material. Such a tobacco material is disclosed, for example, in U.S. Patent No. 5,101,839 to Jakob et al*.* and U.S. Patent Application No. 2010/0186757 to Crooks et al. In addition, the slurry can incorporate granular inorganic material (i.e., calcium carbonate). The slurry is cast unto a paper element of a foil-paper laminate, such as disclosed, for example, in U.S. Patent No. 8,678,013 to Crooks et al. and U.S. Patent No. 7,647,932 to Cantrell et al*,* and the assembled cast sheet product is then dried, for instance by the application of heat (i.e., by heated air, microwave drying, etc.). The paper element may have, for instance, a particular porosity or texture to promote an intimate contact and interaction with the slurry, for instance, over direct contact between the slurry and the foil. However, the exemplary aspect presented herein does not preclude casting the tobacco material (i.e., slurry) directly on a metal foil or other suitable thin film heat conductor. Once such a laminate is cast, the dried cast sheet (i.e., the foil/paper/tobacco material) may be shredded, diced, or otherwise separated into a plurality of cast sheet portion elements, wherein each such element preferably includes a portion of the tobacco material (i.e., the substrate) intimately interacted with a portion of the paper element which, in turn, is in intimate contact with a portion of the foil element of the foil-paper laminate. A plurality of the cast sheet portion elements may then be included in the aerosol-generating element(s) 425 forming the second aerosol generation arrangement 400.

One skilled in the art will appreciate that, in some circumstances, the cast sheet portion elements included in the aerosol-generating element(s) 425 may cooperate to promote improved heat transfer to the tobacco material forming a portion of those cast sheet portion elements or otherwise to abutting elements. More particularly, in some instances, heat transfer from the heating element 240 to the tobacco material included in the aerosol-generating element(s) 425 may be limited past any direct interface therebetween, with the heat-conducting strip forming an additional mechanism for conducting heat from the heating element 240 for heating the outer elements included in the aerosol-generating element(s) 425 and any aerosol-generating element(s) in contact therewith. In aspects including the cast sheet portion elements included in the aerosol-generating element(s) 425, the heat-conductive portions of the foil element associated with the cast sheet portion elements may form, for example, a plurality of additional heat conductive pathways. That is, the cast sheet portion elements used as all or part of the aerosol-generating element(s) 425 may provide additional heat-conductive elements interspersed throughout the aerosol-generating element(s) 425 within the second aerosol generation arrangement 400 to thereby enhance or otherwise improve heat transfer to and between the aerosol-generating elements. In achieving such an aspect, it may be further advantageous to shred or process a substrate material implemented in, for example, the cast tobacco sheet substrate material forming the substrate incorporated within the types of cigarettes commercially marketed under the trade name "Eclipse" by R. J. Reynolds Tobacco Company, as disclosed, e.g., by U.S. Patent No. 5,469.871 to Barnes et al.

The pellets or other elements may have smooth, regular outer shapes (e.g., spheres, cylinders, ovoids, or the like) and/or they may have irregular outer shapes (e.g., shredded pieces, flakes, or the like). The aerosol-generating element(s) 425, discretely or cumulatively, may have a generally cylindrical form within the second aerosol generation arrangement 400, and may in some instances include a collection of about 800 to about 1200 generally spherical beads, each having a mean or nominal diameter of about 0.05 mm to about 4 mm (e.g., about 1 mm³ in volume, in one example), with the beads/pellets cumulatively weighing about 450 mg to about 750 mg (e.g., 600 mg ± 25%, in one example).

**In** one method of preparation, substantially spherical beads or pellets of aerosol-generating elements can be formed by first mixing together the desired composition followed by extrusion of the composition to form an extrudate. The extrudate is then processed in a spheronizer (e.g., such as spheronizers available from Caleva Process Solutions Ltd. or LCI Corporation) to produce variously-sized spheroids that can be processed through a series of screens to provide the desired size range, such as the sizes noted above.

The aerosol-generating elements can be selected so as to have relatively uniform mean diameter or a range of sizes of aerosol-generating elements can be included in the second aerosol generation arrangement 400. Where different size ranges are used in the same device, the differently sized elements can be arranged in a gradient or layers within the second aerosol generation arrangement 400 or the differently sized elements can be randomly mixed within the aerosol generation arrangement 400. Although not bound by any particular theory of operation, using aerosol-generating elements of different sizes in the same aerosol generation arrangement 400 can provide advantageous pressure drop changes in the device and/or provide advantageous sensory characteristics based on the different rates of evaporation provided by the differently sized elements.

Preferably, sufficient beads are loaded into the second aerosol generation arrangement 400 to provide at least about 95 percent of maximum fill, with beads and/or other suitable elements. It is advantageous to avoid large open pockets within the aerosol generation arrangement 400 that could allow air traveling through the aerosol generation arrangement to substantially bypass interaction with the aerosol-generating elements 425.

In some instances, a plurality of forms of the aerosol-generating element(s) 425 may be selected (e.g., aerosol-generating element(s) having different compositions) and each selected form of the aerosol-generating elements then subsequently included in the second aerosol generation arrangement 400. In other instances, the selected forms of the aerosol-generating elements may be combined, prior to inclusion in the second aerosol generation arrangement 400, to produce an aerosol-generating element mixture, and the mixture then subsequently included in the second aerosol generation arrangement 400.

The atomizer or first aerosol generation arrangement 212 and the second aerosol generation arrangement 400 may be physically separate from one another and/or comprise discrete units or segments within the cartridge body 200. In some instances, as shown, those segments may be positioned/disposed so that the downstream end (toward the mouthpiece or mouth-engaging end 220 of the cartridge body 200) of the atomizer or first aerosol generation arrangement 212 is adjacent to the upstream end of the second aerosol generation segment 400 (i.e., the back face of the aerosol-generating element(s) 425). That is, the atomizer or first aerosol generation arrangement 212 and the second aerosol generation segment 400 may be axially aligned in a serial end-to-end relationship, in some instances adjacent to or abutting one another. For example, in some instances, though physically discrete and positioned downstream from the atomizer or first aerosol generation arrangement 212, it may be desirable for the aerosol-generating element(s) 425 of the second aerosol generation arrangement 400 to physically contact the heating element 240 at the downstream end of the atomizer or first aerosol generation arrangement 212. Alternatively, those segments 212, 400 can be slightly spaced apart from one another such that the respective ends or components thereof 240, 425 are not necessarily in physical contact with the other (i.e., to prevent scorching). One skilled in the art will appreciate that, in some aspects, the second aerosol generation arrangement 400 may comprise more than one section or portion of aerosol-generating element(s) 425.

In some instances, an additional segment, spacer element, or separating element (otherwise referred to herein as "a first separating element"), acting as a spacer or screen (see, e.g., element 450 in FIG. 3) may be positioned generally perpendicular to the longitudinal axis of the cartridge body 200, wherein the first separating element 450 may provide for physical separation of those two segments 212, 400 while, in some instances, maintaining a heat conductive relationship therebetween. The first separating element 450 may, in some instances, not be conductive to heat and, in other instances, the first separating element 450 may not be electrically conductive. That is, the first separating element 450 may, but not necessarily, be heat-conductive and/or arranged to conduct heat from the heating element 240 of the atomizer / first aerosol generation arrangement 212 to the second aerosol generation arrangement 400, wherein the aerosol-generating element(s) 425 may be responsive to the heat and/or accompanying first aerosol to form a second aerosol. Further, in some instances, the first separating element 450 may be air permeable or otherwise configured to permit airflow therethrough, such that a first aerosol generated by the atomizer / first aerosol generation arrangement 400 can pass therethrough in the downstream direction. The first separating element 450 may thus also be configured and/or arranged so as to maintain the aerosol-generating element(s) 425 within the second aerosol generation arrangement 400 and separate from the atomizer/first aerosol generation arrangement 212. In still further instances, the first separating element 450 may be configured as a spacer (i.e., extending in a longitudinal direction along the cartridge body 200 so as to define a thickness) for separating the aerosol-generating element(s) 425 from the heating element 240 of the atomizer/first aerosol generation arrangement 212, for example, to minimize or prevent the aerosol-generating element(s) (i.e., beads) 425 from being scorched or burned by the heat from the heating element 240. In some instances, the first separating element 450 may also be configured as an insulator (i.e., not electrically conductive) to prevent short-circuiting of the heating element 240 in the event of contact therebetween.

Typically, the first separating element 450 is generally cylindrical or discoid in shape and of one piece construction, and is air permeable to allow the passage of drawn air through. The first separating element 450 may be heat conductive in nature, so that heat generated by the heating element 240 can be readily transported to the second aerosol generation arrangement 400. The length (thickness) of the first separating element 450 can vary, and typically extends from about less than 1 mm up to about 10 mm. In some instances, the relative longitudinal placement of the first separating element 450 within the outer body 216, spaces the interface of the first separating element 450 with the aerosol-generating element(s) 425 at between about 1 mm and up to about 20 mm (i.e., 7 mm in one example) away from the heating element 240. Typically, the first separating element 450 is comprised of a heat resistant material, such as a porous ceramic, a porous graphite material, a metal (i.e., stainless steel, brass, copper, etc.) mesh or screen, a high temperature-resistant plastic or the like. In some instances, the first separating element 450 may include, for example, longitudinally-extending air passageways formed during design / manufacture, drilled therethrough, or otherwise molded, extruded, printed (i.e., a 3D printed element using a 3D printer), or shaped into the spacer element during manufacture thereof. If desired, the first separating element 450 can incorporate catalytic materials, such as materials incorporating cerium or copper ions or oxides and/or salts of cerium and copper ions. See, for example, U.S. Patent Nos. 8,469,035 and 8,617,263 to Banerjee et al*.* and U. S. Pat. Appl. Pub. No. 2007/0215168 to Banerjee et al.

In instances where the aerosol-generating element(s) 425 may be circumscribed by an insulation layer, a layer of heat conductive material (e.g., a layer or strip comprised of metal foil) may be provided therebetween (not shown). That is, representative aerosol-generating element(s) 425 include a plurality of pellets and/or other appropriate elements that can be circumscribed along its length by a layer of strip of metal foil. A representative metal foil is, for example, aluminum foil having a thickness of about 0.01 mm to about 0.05 mm. Preferably, the metal foil extends along the entire length of the outer co-axial surface of the aerosol-generating element(s) 425; and it may be preferred that the metal foil extends over (i.e., at least partially overlaps) the first separating element 450. The heat conductive material can be provided by means other than the use of metal foil. For example, the layer of metal foil can be replaced by a metal mesh or screen. Alternatively, the metal foil can be replaced by a heat conductive fabric, such as a layer or sheet of graphite fibers or heat conductive ceramic fibers. Alternatively, the heat conductive material can be provided by application of a heat conductive ink, such as a coating of ink or paint that incorporates metal particles, graphite fibers, particles of heat conductive ceramic materials, or the like.

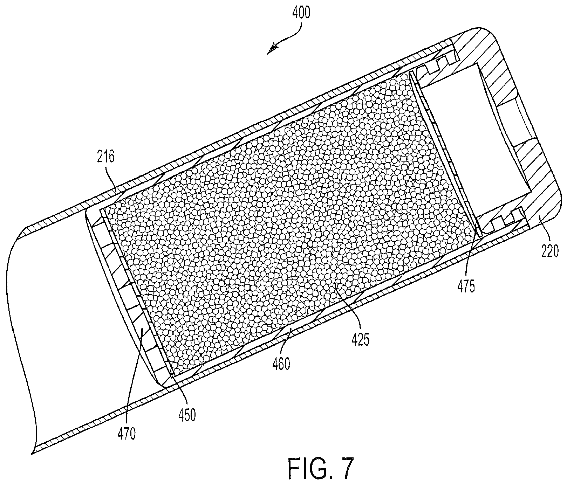

FIG. 7 provides a reference example of a second aerosol generation arrangement 400 positioned within the outer body or tubular member 216 (downstream of the first aerosol generation arrangement 212, which is not shown). As shown, the aerosol-generating elements 425 are placed between separating elements, 450 and 475, which serve to retain the aerosol-generating elements 425 in place and to allow airflow therethrough. As noted previously, the separating elements, 450 and 475, can be porous elements (e.g., mesh screens or perforated metal plates) with pore sizes selected to as to retain the aerosol-generating elements 425 within the second aerosol generation arrangement 400.

As shown, the second aerosol generation arrangement 400 further includes a separate aerosol-generating element housing 460 in the form, for example, of a tubular housing with an open end facing the mouthpiece 220, which as shown, can engage the open end of the tubular housing and can be affixed thereto by a press fit or other known means. The housing 460 can include an end 470 opposite the mouthpiece 220, which as shown, can be perforated to allow airflow therethrough. The housing 460 can be constructed of any suitable material including metal (e.g., stainless steel) or plastic. The separating elements, 450 and 475, can be press fit or otherwise engaged with the housing 460, and the separating element 475 closest to the mouthpiece 220 can be affixed to the mouthpiece if desired. In certain embodiments, the separating elements, 450 and 475, are incorporated into the housing 460 during the molding process that forms the housing. The reference design of FIG. 7 is particularly well-suited for embodiments similar to those of the invention wherein the second aerosol generation arrangement 400 is intended to be permanently affixed to the remainder of the cartridge body 200, rather than separately removable or disposable.

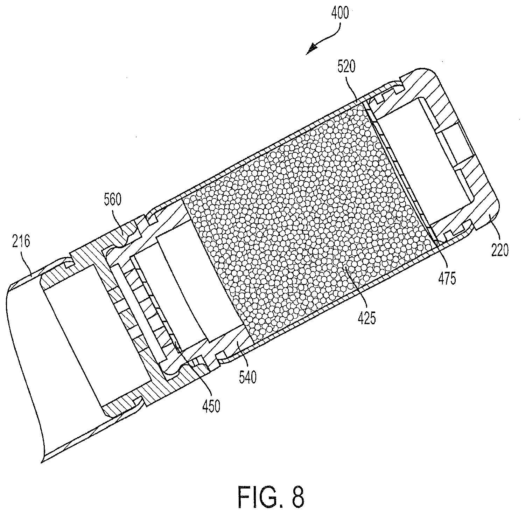

Alternatively, in embodiments where the second aerosol generation arrangement 400 is adapted for removal from the cartridge body 200 as a separate unit, the reference design of FIG. 8 is advantageous. As shown, in the reference design of FIG. 8, the second aerosol generation arrangement 400 is formed as a separate unit with a separate housing body 520, which is attached (e.g., through crimping or other means) to a first connector 540. Together, the housing body 520 and first connector 540 form a cavity for the aerosol-generating elements 425. As with the illustration of FIG. 7, the aerosol-generating elements 425 are placed between separating elements, 450 and 475, which serve to retain the aerosol-generating elements 425 in place and to allow airflow therethrough. Similar to the illustration of FIG. 7, the separating elements, 450 and 475, can be press fit or otherwise engaged with the surrounding portions of the first connector 540 or housing body 520, respectively, and can be incorporated into these surrounding portions during a molding process. The downstream separating element 475 is also optionally affixed to the mouthpiece 220.

The first connector 540 of the second aerosol generation arrangement 400 is adapted for engagement with a second connector 560 that is affixed (e.g., through press fit or other means) to the outer body or tubular member 216 housing the first aerosol generation arrangement 212 (not shown). The second connector 560 has an end facing the first connector 540 that enables the user to removably affix the second aerosol generation arrangement 400 to the cartridge body 200, such as through a threaded engagement or other connection means. As shown, the second connector 560 is porous to allow airflow from the first aerosol generation arrangement 212 to enter the second aerosol generation arrangement 400. The second aerosol generation arrangement 400 of this design is cooperatively engaged with the mouthpiece 220 in a manner similar to FIG. 7.

In some aspects, another spacer element, or another separating element (otherwise referred to herein as "a second separating element"), acting as a spacer or screen (see, e.g., element 475 in FIG. 3) may be positioned generally perpendicular to the longitudinal axis of the cartridge body 200, wherein the second separating element 475 may provide for physical separation of the second aerosol generation arrangement 400 from the mouthpiece or mouth-engaging end 220 of the cartridge body 200. That is, the second separating element 475 may, but not necessarily, be heat-conductive and/or arranged to conduct heat from the second aerosol generation arrangement 400 and through the mouthpiece or mouth-engaging end 220 of the cartridge body 200. However, the second separating element 475 may be air permeable or otherwise configured to permit airflow therethrough, such that a first aerosol generated by the atomizer / first aerosol generation arrangement 212 and/or a second aerosol generated by the second aerosol generation arrangement 400, can pass therethrough in the downstream direction and through the mouthpiece or mouth-engaging end 220 of the cartridge body 200. The second separating element 475 may thus also be configured and/or arranged so as to maintain the aerosol-generating element(s) 425 within the second aerosol generation arrangement 400, without loss of any of the aerosol-generating element(s) through the mouthpiece or mouth-engaging end 220 of the cartridge body 200.

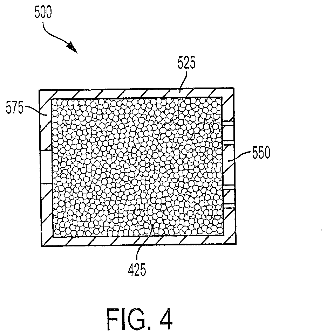

In some aspects, in the alternative to discrete first and second separating elements 450, 475 being implemented in addition to the aerosol-generating element(s) 425, the second aerosol generation arrangement 400 may comprise a cartridge 500 (see, e.g., FIG. 4) having an elongate tubular body 525 and opposed end members 550, 575, wherein each of the end members 550, 575 may be heat-conductive and/or air permeable in a similar manner to the first and second separating elements 450, 475. The elongate tubular body 525 may thus be further configured to receive the aerosol-generating element(s) 425 and to cooperate with the opposed end members 550, 575 to contain the aerosol-generating element(s) 425 therein. The assembled cartridge 500 may thus be configured to be received as a unit (forming the second aerosol generation arrangement 400) by the outer body or tubular member 216 of the cartridge body 200.

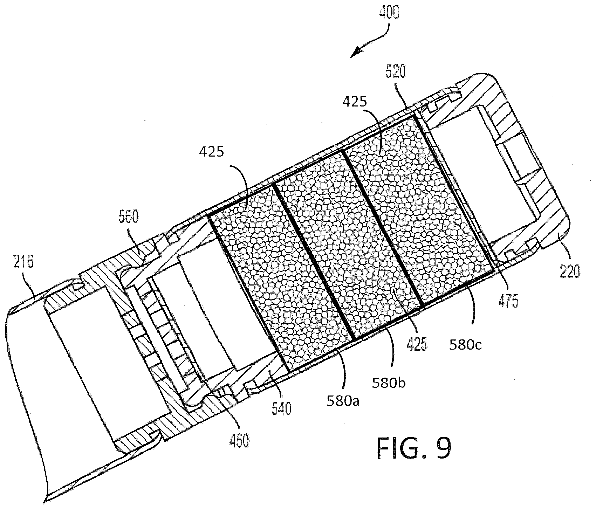

FIG. 9 illustrates an embodiment of the invention wherein the housing body 520 contains multiple, stackable containers, 580a, 580b, 580c, each container housing aerosol-generating elements 425. Each container, e.g., 580a, can include different aerosol-generating elements 425 that provide a different sensory experience. In this manner, the user can stack multiple containers of different types in any desired order to change the sensory experience of the aerosol delivery system 100. Each individual container, 580a, 580b, 580c, can have a similar construction, such as, for example, the general structure set forth in FIG. 4, such that the aerosol-generating elements 425 are housed in a porous-walled container that allows gaseous flow therethrough. The number of stackable containers used can vary and is not limited to the illustrated embodiment of three containers. An exemplary range for the total number of stackable containers is 2 to about 8 (e.g., 2, 3, 4, 5, 6, 7, or 8 containers arranged in stacked form).

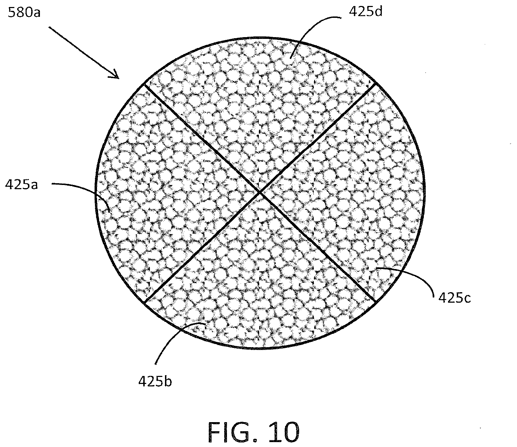

FIG. 10 illustrates an embodiment wherein a stackable container 580a, as shown in FIG. 9, has internal walls dividing the internal compartment into multiple, wedge-shaped compartments so that different aerosol-generating elements 425 (e.g., 425a, 425b, 425c, and 425d) can be placed in separate compartments within the same container. The cross-sectional shape of each subdivided compartment does not have to be wedge-shaped. Additional non-limiting examples of compartment shapes include concentric circles, triangular, or rectangular compartments. The number and size of the subdivided compartments can vary and is not limited to the illustrated embodiment of four compartments. An exemplary range for the total number of compartments is 2 to about 6 (e.g., 2, 3, 4, 5, or 6 compartments). The use of internal walls to divide a compartment into multiple compartments containing aerosol-generating elements 425 is not limited to the stackable container embodiment of FIG. 9. For example, the single compartment set forth in FIG. 8 could also be subdivided in this manner.

In use, the mouthpiece or mouth-engaging end 220 of the cartridge body 200 of the aerosol delivery system 100 is inserted into the mouth of the user. The atomizer/first aerosol generation arrangement 212 is then actuated, for example, by the user drawing (e.g., a suction) on the mouthpiece or mouth-engaging end 220 of the cartridge body 200. The heating element 240 and the liquid transport element 238 are configured so as to be in a heat exchange relationship. That is, the heat generated by the heating element 240 acts to heat the aerosol precursor composition carried by the liquid transport element 238 to produce a first aerosol. The heat generated by the heating element 240 and the first aerosol are then drawn into engagement with and through the second aerosol generation arrangement 400 (i.e., through the aerosol-generating element(s) 425) toward the inhalation hole defined by the mouthpiece or mouth-engaging end 220. In some instances, the heat from the heating element 240 may interact with the aerosol-generating element(s) 425 to generate a second aerosol. The second aerosol may interact or mix with the first aerosol to form a tertiary aerosol, the tertiary aerosol being the aerosol delivered to the user by way of the mouthpiece 220 in response to the draw imparted thereto by the user. In some instances, the interaction between the heat and/or the first aerosol and the aerosol-generating element(s) 425 may cause an enhancement substance to be imparted to the first aerosol so as to produce an enhanced aerosol. For example, a medicament adsorbed on the aerosol-generating element(s) 425 may react with the first aerosol and/or the heat, or otherwise be de-adsorbed from the aerosol-generating element(s) 425 by the first aerosol and/or the heat, and combine with the first aerosol to form the enhanced aerosol. In still other instances, the aerosol-generating element(s) 425 may be configured such that interaction of the first aerosol therewith causes heat to be drawn away from the first aerosol (i.e., cooling of the first aerosol). When appropriately implemented by the user, at least the first aerosol generated by the atomizer 212 and affected by the second aerosol generation arrangement 400 aerosol are generated and drawn into the mouth of the user.

The components of the second aerosol generation arrangement 400 and/or the aerosol-generating element(s) 425 therein can vary. In general, the second aerosol generation arrangement 400 and/or the aerosol-generating element(s) 425 therein may incorporate components that can be vaporized, aerosolized or entrained in air drawn through the aerosol delivery system 100 during use. Most preferably, those components, by themselves or in cooperation with the first aerosol produced by the first aerosol generation arrangement 212, provide sensory and organoleptic effects, such as aroma, flavor, mouthfeel, visible aerosol sensations, and the like. Examples of components of the first and/or second aerosol generation arrangement 212, 400 that are drawn into the mouth of the user during draw include water (e.g., as water vapor), visible or not visible aerosol forming materials (e.g., glycerin), various volatile flavors (e.g., vanillin and menthol), volatile components of tobacco (e.g., nicotine), and the like.

A preferred aerosol-forming material produces an aerosol (whether visible or not) upon the application of sufficient heat thereto, or otherwise through the action of aerosol forming conditions using components of the aerosol delivery system. A preferred aerosol-forming material produces a visible aerosol that can be considered to be "smoke-like." A preferred aerosol-forming material is chemically simple, relative to the chemical nature of the smoke produced by burning tobacco. A preferred visible aerosol-forming material is a polyol, and exemplary preferred aerosol forming materials include glycerin, propylene glycol, and mixtures thereof. If desired, aerosol forming materials can be combined with other liquid materials, such as water. For example, aerosol forming material formulations can incorporate mixtures of glycerin and water, or mixtures of propylene glycol and water. See, for example, the various aerosol forming materials referenced in U.S. Pat. No. 8,678,013 to Crooks et al.

The aerosol forming materials are carried or supported by substrate materials so as to maintain those aerosol materials within the desired region of the smoking article. Exemplary substrate materials, and exemplary formulations incorporating aerosol-forming materials, are set forth in U.S. Pat. No. 4,793,365 to Sensabaugh et al.; U.S. Pat. No. 4,893,639 to White; U.S. Pat. No. 5,099,861 to Clearman et al.; U.S. Pat. No. 5,101,839 to Jakob et al.; U.S. Pat. No. 5,105,836 to Gentry et al.; U.S. Pat. No. 5,159,942 to Brinkley et al.; U.S. Pat. No. 5,203,355 to Clearman et al.; U.S. Pat. No. 5,271,419 to Arzonico et al.; U.S. Pat. No. 5,327,917 to Lekwauwa et al.; U.S. Pat. No. 5,396,911 to Casey, III et al.; U.S. Pat. No. 5,533,530 to Young et al.; U.S. Pat. No. 5,588,446 to Clearman; U.S. Pat. No. 5,598,868 to Jakob et al.; and U.S. Pat. No. 5,715,844 to Young et al.; and U.S. Patent Application Pub. No. 2005/0066986 to Nestor et al. See, also, Chemical and Biological Studies on New Cigarette Prototypes that Heat Instead of Burn Tobacco, R. J. Reynolds Tobacco Company Monograph (1988). Exemplary substrate materials have been incorporated within the types of cigarettes commercially marketed under the trade names "Premier" and "Eclipse" by R. J. Reynolds Tobacco Company.

In some instances, the aerosol delivery system described with reference to FIG. 1 may be used in much the same manner as commercially marketed e-cigarettes. As a result, when smoked, a preferred aerosol delivery system 100 of the types disclosed herein may yield visible mainstream aerosol resulting principally from volatilized components of the first and second aerosol generation arrangements 212, 400, and that visible aerosol resembles in many regards the mainstream tobacco smoke of a traditional type of cigarette that burns tobacco cut filler.

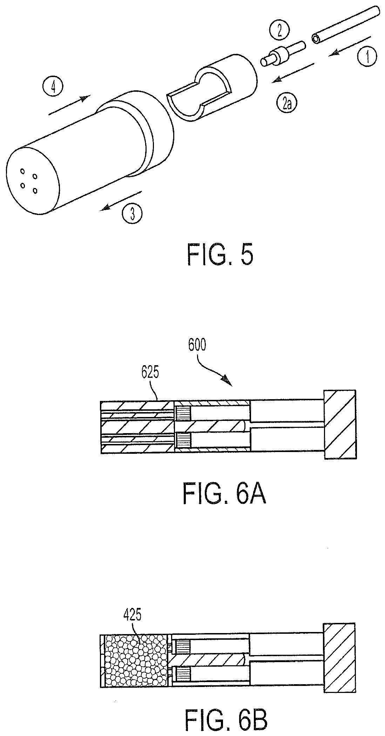

In another example, substantially the entirety of the cartridge body 200 may be formed from one or more carbon materials (see, e.g., FIG. 5), which may provide advantages over other cartridge body configurations disclosed herein in terms of biodegradability and absence of wires. In this regard, the heating element may comprise carbon foam, the reservoir may comprise carbonized fabric, and graphite may be employed to form an electrical connection with the battery and controller. Examples of a carbon-based cartridge body are provided in U.S. Pat. App. Pub. Nos. 2015/0059780 to Davis et al. or 2013/0255702 to Griffith et al. In some instances, the incorporation of the second aerosol generation arrangement disclosed herein may also be applicable to such a carbon-based cartridge body. For example, as shown in FIG. 6A and 6B, the portion 625 (see, e.g., FIG. 6A) of the cartridge element 600 disposed toward the mouthpiece of the cartridge body may be configured or otherwise altered (see, e.g., FIG. 6B) so as to receive one or more of the aerosol-generating element(s) 425 of the types disclosed herein. In the alternative, a pre-assembled cartridge including such aerosol-generating element(s) 425 may be implemented, or the cartridge element 600 and/or the outer body receiving the cartridge element 600 may be configured to receive the first and second separating elements having the aerosol-generating elements therebetween, as otherwise disclosed herein.

Many modifications and other aspects of the disclosures set forth herein will come to mind to one skilled in the art to which these disclosures pertain having the benefit of the teachings presented in the foregoing descriptions and the associated drawings. Although specific terms are employed herein, they are used in a generic and descriptive sense only and not for purposes of limitation.

## Claims

1. An aerosol delivery system (100), comprising:
a control body portion (300) including a first elongate tubular member having opposed ends, and a power source (316) disposed therein;
a cartridge body portion (200) including a second tubular member having opposed first and second ends, the first end being engaged with one of the opposed ends of the control body portion (300), the cartridge body portion (200) further comprising a first aerosol generation arrangement (212) disposed within the second tubular member and configured to operably engage the power source (316) upon engagement between the one of the opposed ends of the control body portion (300) and the first end of the cartridge body portion (200), the second end of the cartridge body portion (200) facing toward a mouth-engaging end (220) of the aerosol delivery system (100); and
a second aerosol generation arrangement (400) disposed between the first aerosol generation arrangement (212) and the mouth-engaging end (220) of the aerosol delivery system (100), the second aerosol generation arrangement (400) being either removably engaged with the cartridge body portion (200) or housed within the second tubular member of the cartridge body portion (200),
wherein the second aerosol generation arrangement (400) further includes a plurality of aerosol-generating elements (425) selected from one or more of flavor-releasing particles, flavor-containing and releasing polymers, granules, co-crystals, powders or microcapsules containing tobacco flavors or other substances in a solid, gelatinous, colloidal or gaseous form, and
**characterised in that** the second aerosol generation arrangement (400) comprises an outer housing body (520) and a plurality of stackable, gas-permeable containers (580a, 580b, 580c) within the outer housing body (520), each container (580a, 580b, 580c) containing a plurality of aerosol-generating elements. (425).

2. The aerosol delivery system (100) of claim 1, further comprising a first separating element (450) disposed within the second tubular member between the first aerosol generation arrangement (212) and the second aerosol generation arrangement (400), the first separating element (450) being one of heat-conductive and air permeable.

3. The aerosol delivery system (100) of claim 2, wherein the first separating element (450) extends along a longitudinal axis between opposed ends so as to define a thickness, the thickness of the first separating element (450) being configured to space the second aerosol generation arrangement (400) from a heating element (240) of the first aerosol generation arrangement (212).

4. The aerosol delivery system (100) of any one of claims 1 to 3, further comprising a second separating element (475) between the second aerosol generation arrangement (400) and the mouth-engaging end (220), the second separating element (475) being one of heat-conductive and air permeable.

5. The aerosol delivery system (100) of any one of claims 1 to 4, wherein the second aerosol generation arrangement (400) comprises a cartridge having an elongate tubular body and opposed end members, each of the end members being one of heat-conductive and air permeable, the elongate tubular body being further configured to receive the plurality of aerosol-generating elements (425) and to cooperate with the opposed end members to contain the plurality of aerosol-generating elements (425) therein, the cartridge being configured to be received by the second tubular member.

6. The aerosol delivery system (100) of any one of claims 1 to 5, wherein the first aerosol generation arrangement (212) comprises a liquid reservoir disposed within the second tubular member and configured to receive an aerosol precursor substance used by the first aerosol generation arrangement (212) to generate a first aerosol.

7. The aerosol delivery system (100) of claim 6, wherein the aerosol precursor substance is one of glycerin, propylene glycol, water, saline, nicotine, organic acids, and combinations thereof.

8. The aerosol delivery system (100) of any one of claims 1 to 7, wherein the first aerosol generation arrangement (212) includes a heating element (240) configured to provide heat for producing a first aerosol, the plurality of aerosol-generating elements (425) being arranged to interact with the heat and the first aerosol, drawn therethrough toward the mouth-engaging end (220), in response to a suction applied to the mouth-engaging end (220).

9. The aerosol delivery system (100) of any one of claims 1 to 8, wherein the aerosol-generating elements (425) comprise one or more of particulate tobacco, a tobacco extract, and nicotine, wherein the nicotine in free base form, salt form, as a complex, or as a solvate.

10. The aerosol delivery system (100) of any one of claims 1 to 9, wherein the aerosol-generating elements (425) further comprise one or more flavorants.

11. The aerosol delivery system (100) of any one of claims 1 to 10, wherein the second aerosol generation arrangement (400) is housed within the second tubular member of the cartridge body portion (200) and includes a plurality of aerosol-generating elements (425) in the form of beads or pellets retained in place by a first air permeable separating element (450) disposed within the second tubular member between the first aerosol generation arrangement (212) and the second aerosol generation arrangement (400) and a second separating element (475) between the second aerosol generation arrangement (400) and the mouth-engaging end (220).

12. The aerosol delivery system (100) of any one of claims 1 to 11, wherein the second aerosol generation arrangement (400) is removably engaged with the cartridge body portion (200).

## Patentansprüche

1. Aerosolabgabesystem (100) umfassend:
einen Steuerkörperteil (300), der ein erstes langgestrecktes rohrförmiges Element mit gegenüberliegenden Enden und eine darin angeordnete Stromquelle (316) enthält;
einen Patronenkörperteil (200), der ein zweites rohrförmiges Element mit gegenüberliegenden ersten und zweiten Enden enthält, wobei das erste Ende mit einem der gegenüberliegenden Enden des Steuerkörperteils (300) in Eingriff steht, wobei der Patronenkörperteil (200) ferner eine erste Aerosolerzeugungsanordnung (212) umfasst, die in dem zweiten rohrförmigen Element angeordnet ist und dafür gestaltet ist, bei Eingriff zwischen dem einen der gegenüberliegenden Enden des Steuerkörperteils (300) und dem ersten Ende des Patronenkörperteils (200) mit der Stromquelle (316) in Funktionseingriff zu kommen, wobei das zweite Ende des Patronenkörperteils (200) einem Mundeingriffsende (220) des Aerosolabgabesystems (100) zugewandt ist; und
eine zweite Aerosolerzeugungsanordnung (400), die zwischen der ersten Aerosolerzeugungsanordnung (212) und dem Mundeingriffsende (220) des Aerosolabgabesystems (100) angeordnet ist, wobei die zweite Aerosolerzeugungsanordnung (400) entweder entfernbar mit dem Patronenkörperteil (200) in Eingriff steht oder in dem zweiten rohrförmigen Element des Patronenkörperteils (200) untergebracht ist,
wobei die zweite Aerosolerzeugungsanordnung (400) ferner eine Vielzahl von Aerosolerzeugungselementen (425) enthalten, die ausgewählt sind aus einem oder mehreren von aromafreisetzenden Partikeln, aromahaltigen und - freisetzenden Polymeren, Granulat, Co-Kristallen, Pulvern oder Mikrokapseln, die Tabakaromen oder andere Stoffe in einer festen, gelatinösen, kolloidalen oder gasförmigen Form enthalten, und
**dadurch gekennzeichnet, dass** die zweite Aerosolerzeugungsanordnung (400) einen äußeren Gehäusekörper (520) und eine Vielzahl von stapelbaren gasdurchlässigen Behältern (580a, 580b, 580c) innerhalb des äußeren Gehäusekörpers (520) umfasst, wobei jeder Behälter (580a, 580b, 580c) eine Vielzahl von Aerosolerzeugungselementen enthält. (425).

2. Aerosolabgabesystem (100) nach Anspruch 1, ferner umfassend ein erstes Trennelement (450), das innerhalb des zweiten rohrförmigen Elements zwischen der ersten Aerosolerzeugungsanordnung (212) und der zweiten Aerosolerzeugungsanordnung (400) angeordnet ist, wobei das erste Trennelement (450) eines von wärmeleitfähig und luftdurchlässig ist.

3. Aerosolabgabesystem (100) nach Anspruch 2, wobei sich das erste Trennelement (450) entlang einer Längsachse zwischen gegenüberliegenden Enden erstreckt, um eine Dicke zu definieren, wobei die Dicke des ersten Trennelements (450) dafür gestaltet ist, die zweite Aerosolerzeugungsanordnung (400) von einem Heizelement (240) der ersten Aerosolerzeugungsanordnung (212) zu beabstanden.

4. Aerosolabgabesystem (100) nach einem der Ansprüche 1 bis 3, ferner umfassend ein zweites Trennelement (475) zwischen der zweiten Aerosolerzeugungsanordnung (400) und dem Mundeingriffsende (220), wobei das zweite Trennelement (475) eines von wärmeleitfähig und luftdurchlässig ist.

5. Aerosolabgabesystem (100) nach einem der Ansprüche 1 bis 4, wobei die zweite Aerosolerzeugungsanordnung (400) eine Patrone mit einem langgestreckten rohrförmigen Körper und gegenüberliegenden Endelementen umfasst, wobei jedes der Endelemente eines von wärmeleitfähig und luftdurchlässig ist, wobei der langgestreckte rohrförmige Körper ferner dafür gestaltet ist, die Vielzahl von Aerosolerzeugungselementen (425) aufzunehmen und mit den gegenüberliegenden Endelementen zusammenzuwirken, um die Vielzahl von Aerosolerzeugungselementen (425) darin zu enthalten, wobei die Patrone dafür gestaltet ist, vom zweiten rohrförmigen Element aufgenommen zu werden.

6. Aerosolabgabesystem (100) nach einem der Ansprüche 1 bis 5, wobei die erste Aerosolerzeugungsanordnung (212) ein Flüssigkeitsreservoir umfasst, das innerhalb des zweiten rohrförmigen Elements angeordnet ist und dafür gestaltet ist, einen Aerosolvorläuferstoff aufzunehmen, der von der ersten Aerosolerzeugungsanordnung (212) verwendet wird, um ein erstes Aerosol zu erzeugen.

7. Aerosolabgabesystem (100) nach Anspruch 6, wobei der Aerosolvorläuferstoff eines von Glycerin, Propylenglycol, Wasser, Kochsalzlösung, Nikotin, organische Säuren und Kombinationen davon ist.

8. Aerosolabgabesystem (100) nach einem der Ansprüche 1 bis 7, wobei die erste Aerosolerzeugungsanordnung (212) ein Heizelement (240) enthält, das dafür gestaltet ist, Wärme zur Herstellung eines ersten Aerosols bereitzustellen, wobei die Vielzahl von Aerosolerzeugungselementen (425) dafür angeordnet ist, mit der Wärme und dem ersten Aerosol, das als Reaktion auf eine an dem Mundeingriffsende (220) angelegte Saugwirkung durch sie hindurch in Richtung zu dem Mundeingriffsende (220) gezogen wird, zu wechselwirken.

9. Aerosolabgabesystem (100) nach einem der Ansprüche 1 bis 8, wobei die Aerosolerzeugungselemente (425) eines oder mehrere von partikelförmigem Tabak, einem Tabakextrakt und Nikotin umfassen, wobei das Nikotin in freier Basenform, Salzform, als Komplex oder als Solvat vorliegt.

10. Aerosolabgabesystem (100) nach einem der Ansprüche 1 bis 9, wobei die Aerosolerzeugungselemente (425) ferner einen oder mehrere Aromastoffe umfassen.

11. Aerosolabgabesystem (100) nach einem der Ansprüche 1 bis 10, wobei die zweite Aerosolerzeugungsanordnung (400) in dem zweiten rohrförmigen Element des Patronenkörperteils (200) untergebracht ist und eine Vielzahl von Aerosolerzeugungselementen (425) in der Form von Kügelchen oder Pellets enthält, die von einem ersten luftdurchlässigen Trennelement (450), das in dem zweiten rohrförmigen Element zwischen der ersten Aerosolerzeugungsanordnung (212) und der zweiten Aerosolerzeugungsanordnung (400) angeordnetist, und einem zweiten Trennelement (475) zwischen der zweiten Aerosolerzeugungsanordnung (400) und dem Mundeingriffsende (220) in Position gehalten werden.

12. Aerosolabgabesystem (100) nach einem der Ansprüche 1 bis 11, wobei die zweite Aerosolerzeugungsanordnung (400) entfernbar mit dem Patronenkörperteil (200) in Eingriff steht.

## Revendications

1. Système de distribution d'aérosol (100), comprenant :
une partie de corps de commande (300) incluant un premier élément tubulaire allongé ayant des extrémités opposées, et une source d'alimentation (316) disposée en son sein ;
une partie de corps de cartouche (200) incluant un second élément tubulaire ayant des première et seconde extrémités opposées, la première extrémité étant mise en prise avec l'une des extrémités opposées de la partie de corps de commande (300), la partie de corps de cartouche (200) comprenant en outre un premier agencement de génération d'aérosol (212) disposé à l'intérieur du second élément tubulaire et configuré pour venir en prise de manière fonctionnelle avec la source d'alimentation (316) lors de la mise en prise entre l'une des extrémités opposées de la partie de corps de commande (300) et la première extrémité de la partie de corps de cartouche (200), la seconde extrémité de la partie de corps de cartouche (200) faisant face à une extrémité de contact avec la bouche (220) du système de distribution d'aérosol (100) ; et
un second agencement de génération d'aérosol (400) disposé entre le premier agencement de génération d'aérosol (212) et l'extrémité de contact avec la bouche (220) du système de distribution d'aérosol (100), le second agencement de génération d'aérosol (400) étant soit mis en prise de manière amovible avec la partie de corps de cartouche (200), soit logé à l'intérieur du second élément tubulaire de la partie de corps de cartouche (200),
dans lequel le second agencement de génération d'aérosol (400) comprend en outre une pluralité d'éléments générateurs d'aérosol (425) sélectionnés parmi une ou plusieurs parmi des particules libérant un arôme, des polymères contenant et libérant un arôme, des granules, des co-cristaux, des poudres ou des microcapsules contenant des arômes de tabac ou d'autres substances sous forme solide, gélatineuse, colloïdale ou gazeuse, et
**caractérisé en ce que** le second agencement de génération d'aérosol (400) comprend un corps de boîtier extérieur (520) et une pluralité de récipients empilables perméables aux gaz (580a, 580b, 580c) à l'intérieur du corps de boîtier extérieur (520), chaque récipient (580a, 580b, 580c) contenant une pluralité d'éléments générateurs d'aérosol. (425).

2. Système de distribution d'aérosol (100) selon la revendication 1, comprenant en outre un premier élément de séparation (450) disposé à l'intérieur du second élément tubulaire entre le premier agencement de génération d'aérosol (212) et le second agencement de génération d'aérosol (400), le premier élément de séparation (450) étant soit conducteur de chaleur, soit perméable à l'air.

3. Système de distribution d'aérosol (100) selon la revendication 2, dans lequel le premier élément de séparation (450) s'étend le long d'un axe longitudinal entre des extrémités opposées de façon à définir une épaisseur, l'épaisseur du premier élément de séparation (450) étant configurée pour espacer le second agencement de génération d'aérosol (400) d'un élément chauffant (240) du premier agencement de génération d'aérosol (212).

4. Système de distribution d'aérosol (100) selon l'une quelconque des revendications 1 à 3, comprenant en outre un second élément de séparation (475) entre le second agencement de génération d'aérosol (400) et l'extrémité de contact avec la bouche (220), le second élément de séparation (475) étant soit conducteur de chaleur, soit perméable à l'air.

5. Système de distribution d'aérosol (100) selon l'une quelconque des revendications 1 à 4, dans lequel le second agencement de génération d'aérosol (400) comprend une cartouche ayant un corps tubulaire allongé et des éléments d'extrémité opposés, chacun des éléments d'extrémité étant soit conducteur de chaleur, soit perméable à l'air, le corps tubulaire allongé étant en outre configuré pour recevoir la pluralité d'éléments de génération d'aérosol (425) et pour coopérer avec les éléments d'extrémité opposés pour contenir la pluralité d'éléments de génération d'aérosol (425) en son sein, la cartouche étant configurée pour être reçue par le second élément tubulaire.

6. Système de distribution d'aérosol (100) selon l'une quelconque des revendications 1 à 5, dans lequel le premier agencement de génération d'aérosol (212) comprend un réservoir de liquide disposé à l'intérieur du second élément tubulaire et configuré pour recevoir une substance précurseur d'aérosol utilisée par le premier agencement de génération d'aérosol (212) pour générer un premier aérosol.

7. Système de distribution d'aérosol (100) selon la revendication 6, dans lequel la substance précurseur d'aérosol est l'un parmi la glycérine, le propylèneglycol, l'eau, une solution saline, la nicotine, des acides organiques et des combinaisons correspondantes.

8. Système de distribution d'aérosol (100) selon l'une quelconque des revendications 1 à 7, dans lequel le premier agencement de génération d'aérosol (212) inclut un élément chauffant (240) configuré pour fournir de la chaleur pour produire un premier aérosol, la pluralité d'éléments de génération d'aérosol (425) étant agencés pour interagir avec la chaleur et le premier aérosol aspiré à travers celui-ci vers l'extrémité de contact avec la bouche (220), en réponse à une aspiration appliquée à l'extrémité de contact avec la bouche (220).

9. Système de distribution d'aérosol (100) selon l'une quelconque des revendications 1 à 8, dans lequel les éléments de génération d'aérosol (425) comprennent l'un ou plusieurs de tabac particulaire, extrait de tabac, et nicotine, dans lequel la nicotine est sous forme de base libre, sous forme de sel, en tant que complexe, ou en tant que solvate.

10. Système de distribution d'aérosol (100) selon l'une quelconque des revendications 1 à 9, dans lequel les éléments de génération d'aérosol (425) comprennent en outre un ou plusieurs arômes.

11. Système de distribution d'aérosol (100) selon l'une quelconque des revendications 1 à 10, dans lequel le second agencement de génération d'aérosol (400) est logé à l'intérieur du second élément tubulaire de la partie de corps de cartouche (200) et inclut une pluralité d'éléments de génération d'aérosol (425) sous la forme de billes ou de pastilles retenues en place par un premier élément de séparation perméable à l'air (450) disposé à l'intérieur du second élément tubulaire entre le premier agencement de génération d'aérosol (212) et le second agencement de génération d'aérosol (400) et un second élément de séparation (475) entre le second agencement de génération d'aérosol (400) et l'extrémité de contact avec la bouche (220).

12. Système de distribution d'aérosol (100) selon l'une quelconque des revendications 1 à 11, dans lequel le second agencement de génération d'aérosol (400) est engagé de manière amovible avec la partie de corps de cartouche (200).
